# EUROPEAN PATENT SPECIFICATION

(11) **EP 2 108 315 B1**
(45) Date of publication and mention of the grant of the patent: **06.09.2017**
(21) Application number: 09161514.6
(22) Date of filing: 12.05.2000
(51) Int. Cl.: A61B 17/00

(54) **Device for closure of body defect openings**
Verschlusseinrichtung zur Reparatur von körperlichen Defektöffnungen
Dipositif visant à fermer une ouverture corporelle

(30) Priority: 13.05.1999 US 134250 P
(43) Date of publication of application: 14.10.2009
(62) Divisional of application: 05027961.1
(73) Proprietor: St. Jude Medical ATG, Inc., Maple Grove, MN 55311 (US)
(72) Inventor: Berg, Todd Allen, Stillwater, MN 55082 (US); Peterson, Alex Alden, Maple Grove, MN 55311 (US); Swanson, William J., St. Paul, MN 55108 (US); Hindrichs, Paul J., Plymouth, MN 55441 (US)
(74) Representative: Gill Jennings & Every LLP

(56) References cited:
- EP-A2- 0 541 063
- WO-A1-97/28744
- WO-A1-98/01086
- WO-A2-98/08462
- DE-A1- 2 822 603
- US-A- 5 853 422

## Description

### Background of the Invention

This invention relates to apparatus for closing intravascular defects and occluding blood flow. In particular, it relates to closing septal defects or holes found between the walls of the four heart chambers and occluding blood flow in sections of a patient's circulatory system.

The heart chambers include left and right atrial chambers in the upper portion and left and right ventricular chambers in the lower portion. Defects in these walls can be formed congenitally or can develop later in life. An atrial septal defect (hereinafter, "ASD") is found between the right and left atrium and a ventricular septal defect (hereinafter, "VSD") is found between the left and right ventricles. The defect allows blood to be shunted between the chambers. causing the heart's pumping action to be inefficient, and creating a risk of embolization (the circulation of an abnormal particle through the bloodstream).

A similar defect is the patent ductus. The patent ductus is a pre-birth opening between the aorta and the pulmonary artery. This opening usually closes naturally, but may remain open and cause oxygenated blood to flow back into the lungs. Another defects are the ductus *arteriosis* and the patent *foramen ovale* (hereinafter, "PFO"). At least fifty percent of stroke patients under 55 years old have a PFO.

Therapeutic treatment of these defects normally requires extensive surgery. For example, treatment typically requires open heart surgery, cardiopulmonary bypass, and stopping of the heart. During treatment, the defect is sewn shut by applying a thin patch over the hole. Less invasive methods for closure of these defects, such as intraluminal transcatheter approached. for example, but provide unreliable delivery and deployment. A transcatheter apparatus has a large delivery profile that limits application of the method to young patients and makes it difficult to match the apparatus to the intracardiac or extracardiac cavity and can result in thrombosis, emboli, or dislodgement due to interference with blood flow.

In WO 97/28744 a plug is disclosed for closure of body defect openings. The plug includes a plurality of fingers having a continuous surface that also have a discontinuous cross-section in a plane perpendicular to the central axis of the plug. This fingers are formed by a plurality of resilient, wire like elements.

It is also known that septal holes cause strokes by shunting clots from the right atrium to the left atrium. From the left atrium, a clot can go to the brain. Some holes are asymptomatic and should still be closed to prevent future stroke. Patients having asymptomatic defects would benefit from a low-invasive and reliable treatment apparatus and method.

In addition to the treatment of septal defects, it is often desirable to occlude blood flow in a section of the circulatory system. Occlusion can control internal bleeding and buffer pressure in the vicinity of an aneurysm.

Therefore. it would be desirable to provide apparatus and methods for treating septal defects, such as ASD, VSD, and PFO, that function at least as well as the proven surgical thin sewn patch, but which are less invasive.

It would also be desirable to provide reliable apparatus and methods for delivery of intraluminal transcatheters and deployment of septal defect devices and plugs.

It would be more desirable to provide these apparatus and methods such that the delivery profile is small and such that they can be used to treat patients of a wide range of ages.

It would be further desirable to provide septal defect devices and occluding plugs that can be properly matched to the intracardiac or extracardiac cavity.

It would be still further desirable to provide apparatus and methods for percutaneous delivery and deployment of occlusion devices for blocking blood flow in various sections of the circulatory system.

### Summary of the Invention

Therefore, it is an object of the invention to provide apparatus for treating septal defects, such as ASD, VSD, and PFO, that function as well as the proven surgical thin sewn patch, but which are less invasive.

Additionally, it is an object of the invention to provide these apparatus such that the delivery profile is small and such that they can be used to treat patients of having a wide range of ages.

It is a further object of the invention to provide septal defect devices and occluding plugs that can be properly matched to the intracardiac or extracardiac cavity.

It is a still further object of the invention to provide apparatus for percutaneous delivery and deployment of occlusion devices for blocking blood flow in various sections of the circulatory system.

In accordance with one aspect of the present invention as defined in claim 1 a plug is provided for closing an aperture in a wall of a patient's body cavity. The plug includes: a frame that has a central axis; a first plurality of fingers configured to engage an interior surface of the body cavity wall; a second plurality of fingers that are attached to the first plurality and are configured to engage an exterior surface of the body cavity wall; and a plugging structure. The fingers can be positioned substantially circumferentially about the central axis. The plugging structure is attached to the frame and spans the aperture when the plug is in position. Furthermore, cross-sections of the frame that lie in a plane substantially perpendicular to the central axis are substantially discontinuous in order to enable the plug, and particularly the frame, to conform to the perimeter, or contour. of the aperture.

### Brief Description of the Drawings

The above and other objects and advantages of the invention will be apparent upon consideration of the following detailed description, taken in conjunction with the accompanying drawings, in which like reference characters refer to like parts throughout, and in which:
FIG. 1 is a plan view of a plug for plugging a septal defect in accordance with this invention ;
FIG. 1A is a partial side elevational view along direction 1A-1A of FIG. 1 in accordance with the principles of this invention ;
FIG. 2 is a side elevational view of a finger of a plug in accordance with this invention ;
FIG. 3 is a side elevational view of another finger of a plug in accordance with this invention;
FIG. 4 is a side elevational view of yet another finger of a plug in accordance with this invention ;
FIG. 5 is a side elevational view of still another finger of a plug in accordance with this invention ;
FIG. 6 is a plan view of another plug for plugging a septal defect in accordance with this invention;
FIG. 7 is a side elevational view of yet another finger of a plug in accordance with this invention;
FIG. 8 is a plan view of yet another plug for plugging a septal defect in accordance with this invention;
FIG. 9 is a plan view of yet another plug for plugging a septal defect in accordance with this invention;
FIG. 10 is a cross-sectional view of a plug for plugging a septal defect disposed within a delivery device in accordance with this invention;
FIG. 11 is a cross-sectional view of the plug shown in FIG. 10 when the plug is partially deployed in the septal defect in accordance with this invention;
FIG. 12 is a cross-sectional view of the plug shown in FIG. 10 and 11 when the plug is fully deployed in the septal defect in accordance with this invention ;
FIG 13 is a cross-sectional view of a plug similar to the one shown in FIGS. 10-12 when the plug is fully deployed showing the delivery path of a delivery device in accordance with this invention ;
FIG. 14 is a cross-sectional view of the plug shown in FIG. 13 for plugging a septal defect when the plug is fully deployed and the delivery device has been retracted from the heart in accordance with this invention;
FIG. 15 is an elevational view of the plug shown in FIGS. 13 and 14 taken along line 15-15 of FIG. 14 in accordance with this invention;
FIG. 16 is a plan view of yet another plug for plugging a septal defect in accordance with this invention;
FIG. 17 is a plan view of the plug shown in FIG. 16 from the opposite side in accordance with this invention ;
FIG. 18 is a cross-sectional view of the plug shown in FIGS. 16 and 17 when the plug is fully deployed in the septal defect in accordance with this invention;
FIG. 19 is a plan view of the plug shown in FIGS. 16-18 taken from line 19-19 of FIG. 18 in accordance with this invention ;
FIG. 20 is a partial elevational view of an unrolled frame for an illustrative plug not in accordance with this invention;
FIG. 21 is a perspective view of the of the frame shown in FIG. 21 with ends attached (showing only a single finger at each axial end of the frame) not in accordance with this invention ;
FIG. 22 is a cross-sectional view of the frame shown in FIGS. 20 and 21 with ends attached not in accordance with this invention;
FIG. 23 is an elevational view of the frame shown in FIG. 22 not in accordance with this invention ;
FIG. 24 is a perspective view of the frame shown in FIG. 21 with an attached plugging structure to form a plug not in accordance with this invention;
FIG. 25 is an elevational view of the plug shown in FIG. 24 (showing a number of forward facing fingers) after the fingers have been bent into the wall-engaging position not in accordance with this invention;
FIG. 26 is a partial elevational view of an unrolled frame with a plugging structure not in accordance with this invention;
FIG. 27 is an elevational view of another unrolled frame for a plug not in accordance with this invention;
FIG. 28 is an elevational view of another plug mounted on a delivery balloon not in accordance with this invention;
FIG. 29 is a cross-sectional view taken of the plug shown in FIG. 28 in position for deployment in a defect not in accordance with this invention;
FIG. 30 is a cross-sectional view of the plug shown in FIGS. 28 and 29 when the plug is deployed in the defect not in accordance with this invention;
FIG. 31 is a cross-sectional view of the plug shown in FIGS. 28-30 in position for deployment showing the delivery path of a delivery device not in accordance with this invention;
FIG. 32 is a cross-sectional view of yet another plug for plugging a septal defect when the plug is fully deployed in the septal defect not in accordance with this invention;
FIG. 33 is a partial elevational view of an unrolled frame for an occlusion device (i.e., a plug) not in accordance with this invention;
FIG. 34 is an elevational view of another occlusion device with barbs mounted on a delivery balloon not in accordance with this invention;
FIG. 35 is a partial elevational view of another occlusion device for occluding a section of a blood vessel not in accordance with this invention; and
FIG. 36 is a cross-sectional view of the occlusion device shown in FIG. 35 deployed in a blood vessel not in accordance with this invention.

### Detailed Description of the Preferred Embodiments

The invention provides apparatus for preventing the flow of body fluids through apertures in body cavity walls and through a patien-t's body tubing, such as a blood vessel. The apparatus can be a plug that is installed in the patient's body using an intraluminal catheter. The plug has a (1) frame that conforms to the walls of an aperture (e.g., a defect) and (2) a plugging structure (e.g., a patch) that prevents the flow of fluid. Although the plug can be installed in a variety of types of body tissues to prevent the flow of body fluid, only embodiments of the invention related to preventing the flow of blood through passageways in the circulatory system will be illustrated herein.

In one embodiment, a plug is provided for closing an aperture or hole in a septal wall of a patient's heart, for example a PFO. The plug has a frame, two pluralities of fingers attached to axial ends of the frame and to each other, and a plugging structure attached thereto. The pluralities of fingers can be integral with the frame and formed from a unitary body. During operation, one plurality of fingers engages an interior surface of the wall and the other set of fingers engages the opposite, or exterior, surface. The plugging structure is supported by the frame and spans the aperture (e.g., defect) to prevent the flow of blood there through.

The fingers are preferably positioned substantially circumferentially (i.e., peripherally) about the plug's central axis, which passes through the frame. The fingers can extend radially away or along the axis. The fingers have ends that are radially proximal to the central axis and which generally define a substantially round or elliptical broken cross-section in a plane substantially perpendicular to the central axis.

Preferably, any cross section of the frame that lies in a plane substantially perpendicular to the axis is substantially discontinuous. This allows the frame to contract and expand radially as necessary for insertion into a delivery device, placement in an aperture, and conformation to the walls of the aperture. The frame itself can comprise an elastic material, such as nitinol. Alternatively, the frame can comprise a plastically deforming material, such as stainless steel. The elastic and plastic embodiments may be delivered differently.

When the plug is inside the patient's body, medical scanners can be used to assist and confirm plug placement and to evaluate the integrity of a plug after it has been in use for an extended period of time. The frame may be equipped with one or more marker structures, which can be radiopaque, to help identify, locate, and orient the plug using images produced with, for example, X-rays, CT scans, ultrasound, and echo techniques

Marker structures can be provided in a variety of forms. For example, a marker structure can be in the form of a marker band made from a radiopaque material that is crimped onto an end portion of a finger. Alternatively, a marker structure can be a rivet that is inserted and locked into a ring or hole in a finger. If marker structures are used, they can be provided on any number of fingers.

Other structures can be present on the fingers of the plug to facilitate its delivery. For example, a finger can be provided with a retention device receptacle. A retention device reciprocates within a delivery sleeve or catheter and engages the fingers while the plug is inserted in the delivery sleeve so that the plug can be reciprocated within the sleeve and shifted into position in the aperture, such as a PFO. Once in position, the retention device can release the fingers so that the fingers spring into engagement with the aperture wall.

In one form, the frame of the plug is insertable into a delivery tube by extending the fingers in a direction substantially parallel to the central axis. The retention device itself has fingers or locking pins that engage the retention device receptacles that reside on the ends of the frame fingers. Retention device receptacle include, but are not limited to, locking pin apertures and nose cone covers.

Fingers may have a variety of designs that are tailored to optimize plug security for the shape and tissue characteristics of a given PFO. For example, fingers can have pointed ends, barbs, or curved portions. In one embodiment, fingers can be curved toward a plane that is perpendicular to the central axis and that passes substantially between the two pluralities of fingers. Fingers can be of substantially similar length or substantially different lengths. Any finger can have different flexural stiffness at different points along its length. One way to accomplish differential flexural stiffness of a finger is to provide a finger having a different thickness or a different width at different points along its length. Alternatively, both the finger thickness and the finger width can vary along the length of a given finger, if desired.

In certain cases, the force applied by a finger to the septum wall can be distributed to minimize stress concentration in the wall. In that case, the plug can be provided with an elastic web supported between adjacent fingers. The web, for example, can include silicone.

The plugging structure that occludes the PFO can be attached to or supported by the frame at proximal or distal ends of the fingers. In either case, the fingers' ends can be provided with support structures with which the plugging structure can be affixed.

In one embodiment, the plugging structure is made from an elastic material and can contract and expand as the frame contracts and expands (e.g., during delivery and deployment). One material that can be used to make the plugging structure is polyester (such as the material sold under the trademark DACRON® by E.I. du Pont de Nemours & Company of Wilmington, Delaware.)

The plugging structure can also be made from cloth and be folded and unfolded as the plug is contracted and expanded as may be necessary for its installation in the defect. In either the elastic or cloth embodiments of the plugging structure, the plugging structure can be attached (e.g., sewn) directly to the frame. The plugging structure can have a guide wire aperture through which a guide wire can pass. If the guide wire is inserted into the patient prior to plug delivery, the guide wire can be used to guide the plug into place in the PFO. The guide wire aperture can be designed to substantially self close after the guide wire is removed from the guide wire aperture. The self-closing feature can be achieved by making the diameter of a guide wire aperture in the relaxed state (i.e., without the wire) small enough to induce clotting and close off blood flow.

In an exemplary form, the plug has a perforated tubular portion that forms a longitudinal passage. Fingers extend from each of the two axial ends of the perforated tubular portion and may be provided in a variety of configurations and made from a variety of materials. Any of the features discussed above may also be included. Like the frames discussed above, the perforated tubular portion is discontinuous along any cross section taken in a plane perpendicular to its longitudinal axis. This feature permits the perforated tubular portion to contract and expand radially and longitudinally for delivery, deployment, and conformation to the walls adjacent a PFO, for example. Similarly, the plurality of fingers may be discontinuous along a cross section taken in a plane perpendicular to the longitudinal axis to allow such contraction and expansion as well.

The exemplary plugging structure can be supported directly or indirectly by the perforated tubular portion. For example, the plugging structure can be attached directly to the perforated tubular portion or to elements of the structure (such as tabs, bosses, extension, or loops). Alternatively, the plugging structure can be attached via interceding support structures (such as attachment rings, clips, or loops) that connect the tubular portion to the plugging structures.

The exemplary perforated tubular portion preferably contracts longitudinally as it expands radially. The tubular portion can be made of a material that deforms plastically or elastically. A plastically deforming material can be, for example, stainless steel or tantalum. The plug is installed by positioning the plug in the aperture of the PFO and expanding a balloon inside the plug to at least partially conform the perforated tubular portion to the perimeter of the aperture. The perforations allow the plug to contract longitudinally in response to the radial expansion. The longitudinal contraction causes the fingers to engage opposing sides of the wall.

According to another form, the exemplary occlusion plug may have a perforated tubular portion for occluding a blood vessel. This type of plug may be desirable to prevent blood flow near a damaged portion of the vessel (e.g., aneurysm). The occlusion plug can be plastically or elastically deformable.

In the exemplary plastic form, the occlusion plug may be installed in the blood vessel by expanding a balloon in a longitudinal passageway of the perforated tubular portion. The expansion of the balloon causes the plug to expand radially and contract longitudinally. This expansion causes the outer surface of the perforated tubular portion to conform to the inner surface of the lumen of the blood vessel. The expansion also causes the fingers at the end of the perforated tubular portion to engage the inner surface of the wall as they are driven radially outward from the longitudinal axis and drawn longitudinally toward the perforated tubular portion.

The fingers of the exemplary occlusion plug preferably extend from only one axial end of the perforated tubular portion. The configuration of the fingers, the structures associated with the fingers, the perforated tubular portion, and the relation of the fingers to the perforated tubular portion are similar to those described above in connection with the PFO plug.

The exemplary plugs may be used in methods for preventing the flow of body fluids through apertures in body cavity walls. For simplicity plugging PFO's alone will be discussed. In a preferred exemplary method , a plug that is at least partially made from an elastic material and is conformable to a defect, such as any of those plastic plugs described above having two opposing pluralities of fingers, is positioned at the defect, conformed to the perimeter of the defect and secured thereto.

In order to position the plug in the PFO, a delivery structure with a sleeve may be provided. During the process of positioning the plug, the plug fingers are extended in a direction that is substantially parallel to the central axis of the plug while the plug is inserted into the sleeve of the delivery structure. A retention device inside the sleeve engages at least some of the extended fingers at the finger ends. The retention device can use locking pins, hooks, or any other means to retain the plug inside the sleeve. The retention elements permit an operator to reciprocate the plug longitudinally with respect to the sleeve and to shift the plug out from the end of the sleeve. The sleeve can be inserted like a catheter through an insertion aperture in a patient's body tissue. The sleeve can then be passed through the patient's internal body tubing or other body structures until the end of the sleeve is positioned within or adjacent the PFO for plug delivery.

Once the end of the sleeve is near or within the PFO, the delivery structure can be shifted relative to the plug and the PFO, thereby removing the delivery structure from the PFO. The plug, however, extends through the PFO and the plug fingers extend outward, preferably radially, from the central axis of the plug on opposite sides of the wall in which the PFO resides. This causes the plug fingers to engage the wall and the plugging structure to substantially occlude the PFO.

Preferably, releasing the plug within the PFO allows the plug to expand elastically inside the PFO until the plug substantially conforms to the inner rim or perimeter of the PFO. In one exemplary methods, the plug is allowed to elastically contract along the central axis of the plug while it expands radially. This longitudinal contraction causes the fingers to engage opposite sides of the same wall of the body cavity. A benefit of this approach is that the plug will center itself with respect to the wall in a direction along the plug's central axis (or along the longitudinal passage of the plug).

According to another exemplary method, a plastically deformable plug can be inserted in a PFO using a balloon. A conformable plug, such as any of those plastically deformable plugs described above, is positioned in the PFO, conformed to the perimeter of the PFO, and then secured thereto. The positioning can be achieved by inserting a delivery balloon into the tubular portion of the plug and delivering both through a patient's body tissue, (e.g., through an insertion aperture and blood vessels), to the PFO. Then, the balloon, which supports the plug, is moved through the patient's body until the plug is appropriately positioned in the aperture -- such that one plurality of fingers is situated on each side of the wall.

The plug conforms to the PFO when the balloon is expanded. This causes the fingers to engage on both sides of the septcal wall. The tubular portion of the plug radially enlarges and conforms to the perimeter of the aperture and the plugging structure occludes the aperture. When a plug (such as any of those described above) is used according to this method, the balloon expansion plastically deforms the tubular portion. This expansion automatically causes the tubular portion to contract in the direction parallel to the central axis of the plug. (It will be appreciated that the tubular portion could be annular or have a ring-like arrangement of tabs or other elements). The axial contraction causes the plug to substantially center itself with respect to the wall and drives the fingers into both sides of the wall.

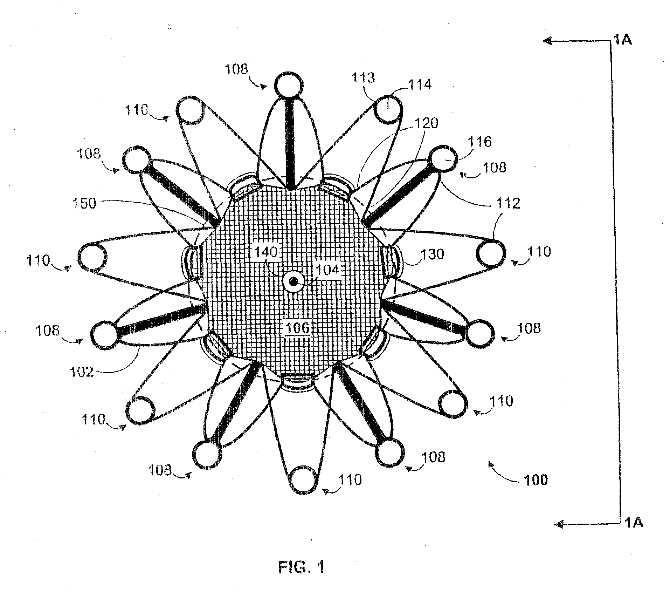

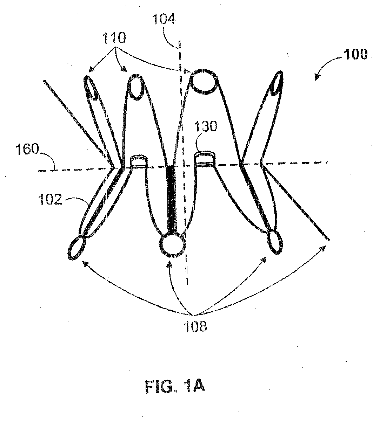

A number apparatus, with several variations, are shown in FIGS. 1-36.

FIG. 1 shows plug 100 for closing an aperture, such as an ASD, a VSD, or a PFO, in a wall of a patient's body cavity. Frame 102 can be made from an elastic material, such as nickel titantium (hereinafter, "nitinol," available, for example, from Shape Memory Applications, of Santa Clara, California). The elastic nature of frame 102 allows frame 102 to radially contract sufficiently to allow it to be inserted into an aperture and subsequently radially expand to conform to the inner perimeter of the aperture. Other elastic materials can also be used to construct the frame and could be used in combination with other non-elastic materials. Radial expandability is facilitated by constructing the frame such that any cross section perpendicular to its central axis is discontinuous.

Frame 102 has central axis 104 and supports plugging structure 106. Frame 102 includes first plurality of fingers 108 and second plurality of fingers 110. Fingers 108 are integral with fingers 110. Fingers 108 and 110 have proximal ends 120 that are near central axis 104 and remote ends 112 that are near the radially outer portions of frame 102.

Proximal ends 120 can be used to support plugging structure 106 directly, or they can be equipped with support structures 130 for supporting plugging structure 106. Alternatively, remote ends 112 can support plugging structure 106. Remote ends 112 can also be equipped with support structures 113 for supporting plugging structure 106, and, as discussed more fully below, marker devices. The ends can also be adapted to engage a retention device during plug installation. In FIG. 1, each support structure 113 has aperture 114 to which a plugging structure can be sewn or otherwise attached.

Plugging structure 106 can be made from an elastic material. Plugging structure 106 can also be folded and unfolded to allow frame 102 to deform during insertion into the aperture. A foldable and unfoldable plugging structure can be either elastic or non-elastic and may include a cloth or polymeric material. In one embodiment, plugging structure 106 is made of polyester.

Plugging structure 106 can include guide wire aperture 140 for insertion of a guide wire (not shown) during installation of the plug in a patient. Guide wire aperture 140 may be self-closing after the removal of a guide wire. In elastic embodiments, the self closing feature may be effected by the elasticity of plugging structure 106. In cloth embodiments, which may or may not be elastic, the woven fibers under tension from frame 102 can automatically close guide wire aperture 140.

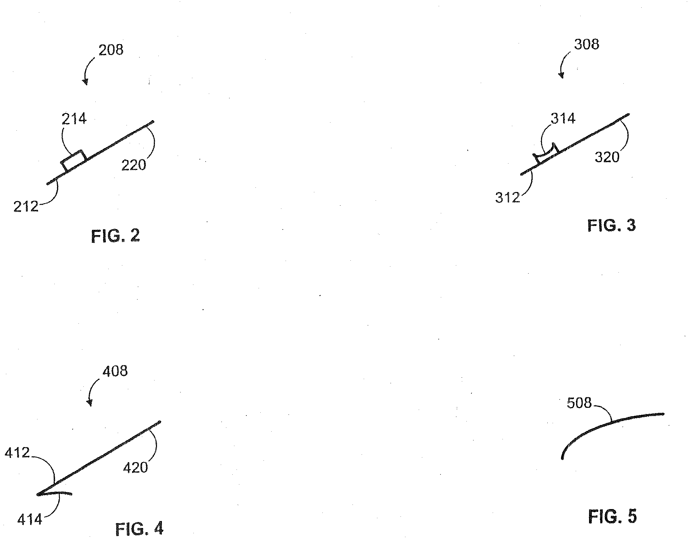

Remote ends 112 can also be provided with retention device receptacles for engaging a retention device that is part of a system for delivering the plug to an aperture in a wall. Finger aperture 114 can be used as a retention device receptacle. A nose cone cover (e.g., cover 314, shown in FIG. 3). is an alternative to a retention device receptacle. The delivery system is discussed below.

As shown in FIG. 1, fingers 108 and 110 can extend substantially radially away from central axis 104, even though some of those fingers may have tangential or spiral components and may not conform to a radial pattern. One or more of fingers 108 and 110 may also contain marker structures, such as a marker band. FIG. 1A shows a partial side view of plug 100 with fingers 108 and 110 in an intermediate configuration without plugging structure 106. For illustrative purposes, only a small number of fingers are shown in FIG. 1A. In an intermediate configuration, fingers are neither parallel nor perpendicular to central axis 104. As can be seen from FIG. 1A, cross-section 160, which is perpendicular to central axis 104 and passes through the medial section of plug 100, is discontinuous.

FIGS. 2-5 show different features that can be incorporated into a finger. FIG. 2, for example, shows a side view of finger 208, having proximal end 220 and remote end 212, with rivet 214 mounted thereto. Rivet 214 can be mounted on a finger such that the rivet head engages the patient's heart wall or such that they face toward the heart cavity. FIG. 3 shows finger 308, having proximal end 320 and distal end 312, with nose cone cover 314. FIG. 4 shows finger 408, having proximal end 420 and distal end 412, with barb 414. FIG. 5 shows finger 508 which is curved. If a plug has two sets of fingers (as shown in FIG. 1), the fingers of each set may be curved toward each other.

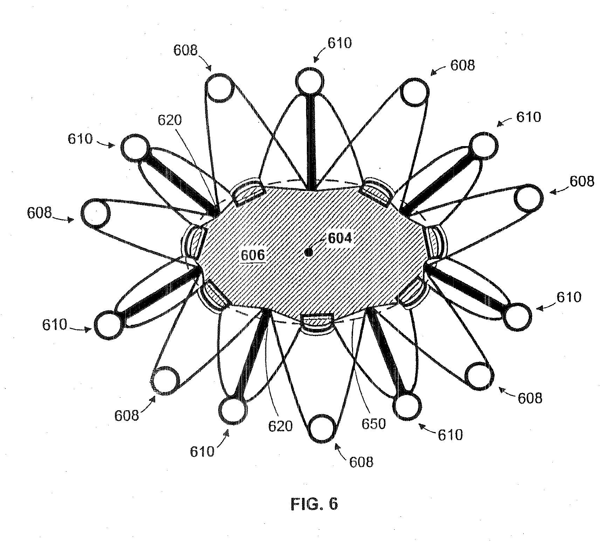

FIG. 6 shows another embodiment of a plug constructed in accordance with this invention in which proximal ends 620 of fingers 608 and 610 define substantially elliptical cross section 650. Elliptical cross section 650 is in contrast to round cross-section 150 shown in FIG. 1. As shown in FIG. 6, central axis 604 passes near the center point of the ellipse plugging structure 606, which is supported by proximal ends 620, has a substantially elliptical shape and could have a guide wire aperture (not shown), if desired.

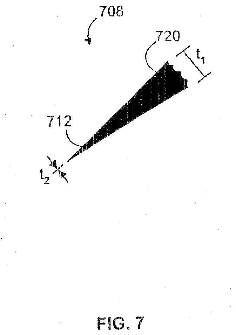

FIG. 7 shows an example of finger 708, which has proximal end 720 and distal end 712. Finger 708 tapers from thickness t₁ at proximal end 720 to lesser thickness t₂ at end 712 (e.g., remote from the plug's central axis). Conversely, finger 708 may be thicker at the remote end and thinner at the proximal end. Thus, a finger, such as finger 708, can have a resulting flexural stiffness that varies along its length. Fingers of varying width, such as the fingers shown in FIG. 1, can also have flexural stiffnesses that vary along their lengths regardless of variations in thickness by varying composition along the length.

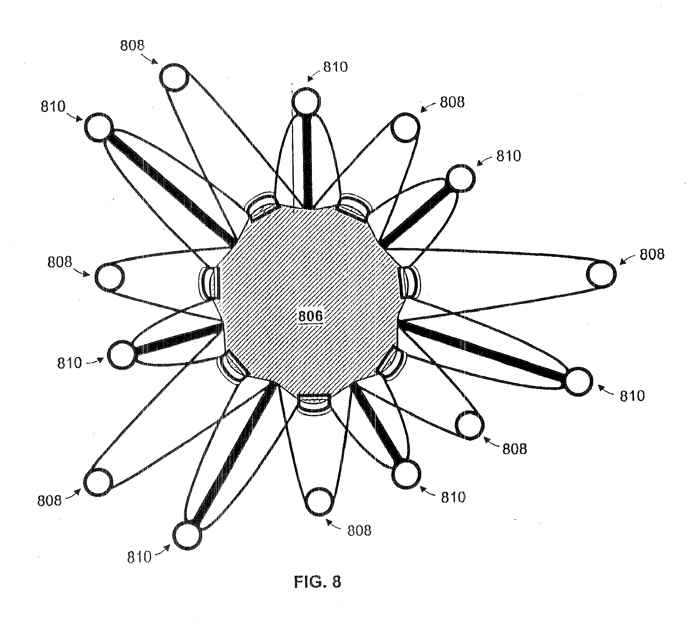

FIG. 8 shows another illustrative embodiment of a plug constructed according to this invention in which fingers 808 and 810 have lengths that vary with respect to each other. Although fingers 808 and 810 have different lengths, it will be appreciated that plugging structure 806 can have a substantially circular, or any other convenient, shape.

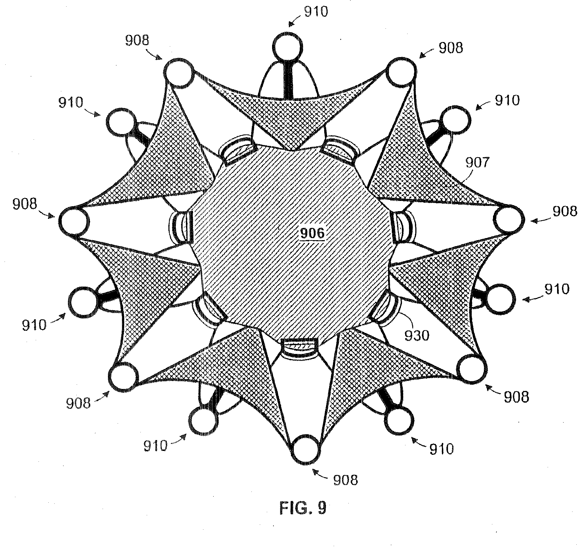

FIG. 9 shows another alternative embodiment of a plug constructed according to this invention in which elastic web 907 spans between adjacent fingers of a plurality of fingers 908. The web can be made from any elastic material, including silicone. Plugging structure 906 is supported by proximal ends of fingers 908 and 910, and can be supported by support structures 930.

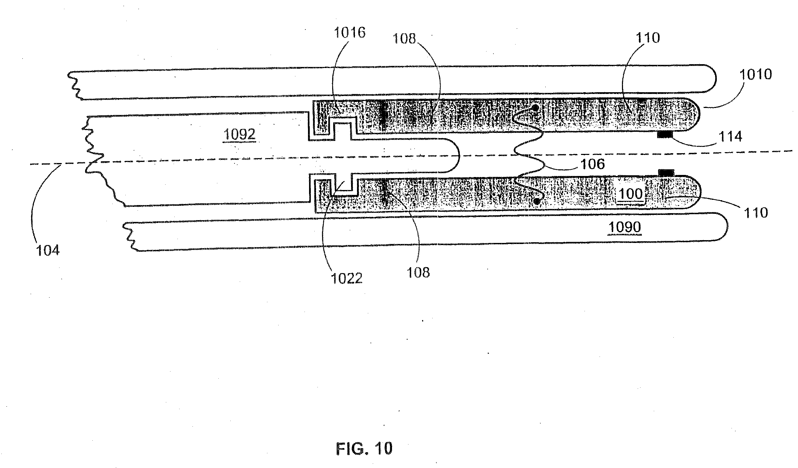

FIG. 10 shows how a plug, such as plug 100, can be installed via a delivery device, such as delivery catheter 1090. Plug 100 is inserted into delivery catheter 1090 by orienting fingers 108 and 110 in a direction that is substantially parallel to central axis 104. Next, plug 100 is passed through to distal opening 1010. Optional marker rivets 114 are shown in FIG. 10. Although it will be appreciated that plugging structure 1006 can be any convenient type, plugging structure 1006 is shown as a folded plugging structure. Retention device receptacles 1016 are engaged with locking pins 1022 of retention device 1092.

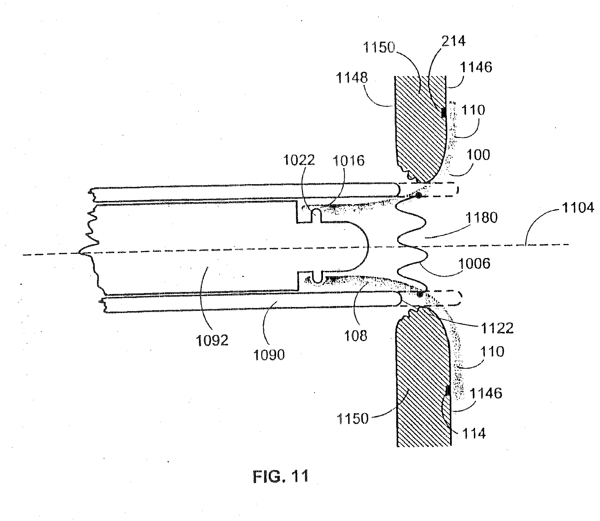

Once plug 100 is positioned near distal opening 1010, plug 100 can be inserted into aperture 1180 in wall 1150 of a patient's body cavity, as shown in FIG. 11. Initially, end 1122 of delivery catheter 1090 is positioned within aperture 1180 (indicated by a dashed line). Delivery catheter 1090 is then partially reciprocated away from aperture 1180 along axis 1104 and with respect to retention device 1092 (indicated by solid line). This forces fingers 110 out of delivery catheter 1090 and causes fingers 110 to spring out radially away from central axis 1104, thereby causing fingers 110 to engage side 1146 of wall 1150. At this stage, fingers 110 conform to the wall and perimeter of aperture 1180. Although FIG. 11 shows markers 114 attached to fingers 110 so they face wall 1050, it will be appreciated that these markers could also be located on the opposite side of these fingers.

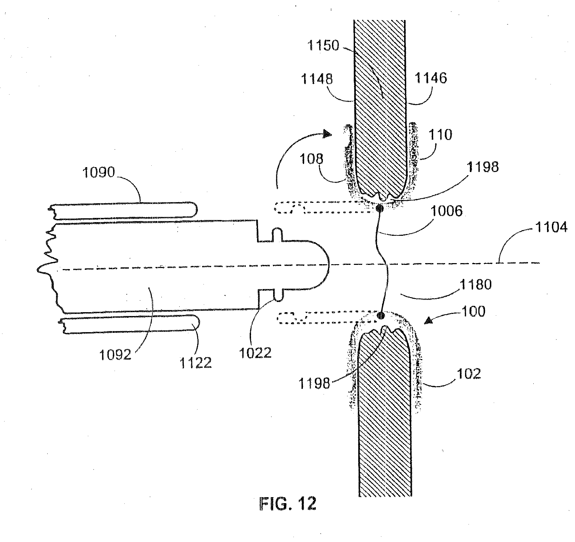

After catheter 1090 is partially reciprocated as shown in FIG. 11, catheter may be further reciprocated as shown in FIG. 12. End 1122 is withdrawn past locking pins 1022 and retention device receptacles 1016. This allows fingers 108 to spring out radially away from central axis 1104 (from a position indicated by the dashed lines) and engage patient's cavity wall 1150 at surface 1148. As shown in FIG. 12, foldable plugging structure 1006 at least partially unfolds to span aperture 1180.

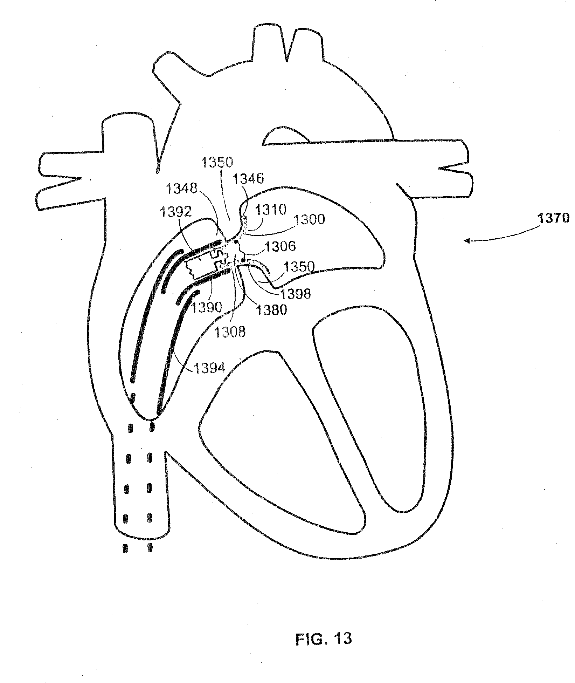

FIG. 13 shows plug 1300 partially installed in aperture 1380 in wall 1350 of heart 1370. Plug 1300 of FIG. 13 corresponds roughly to plug 100 of FIG. 11. Delivery catheter 1390 and retention device 1392 are guided to wall 1350 by delivery guide 1394. At this stage of the installation, fingers 1310 are deployed and engaged with surface 1346 of wall 1350. Fingers 1308 remain in delivery catheter 1390. Plugging structure 1306 is positioned inside aperture 1380 and is ready to conform to perimeter 1398 of aperture 1380 when the remainder of plug 1300 is released from retention device 1392.

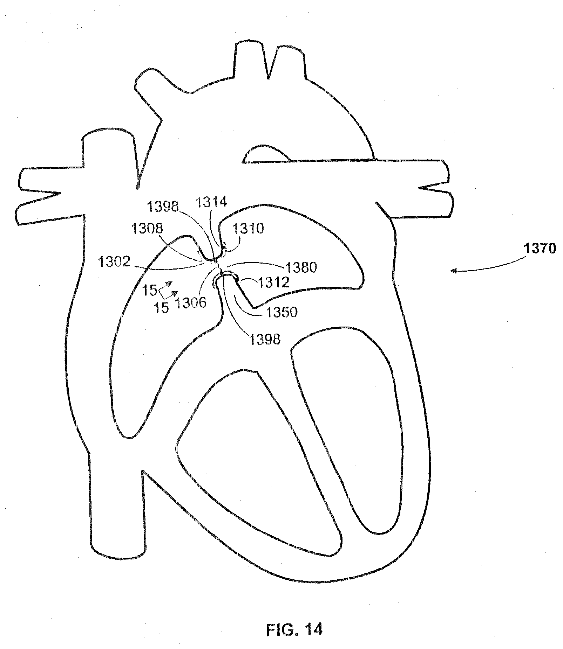

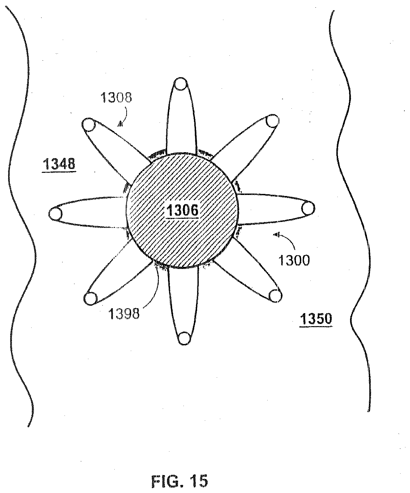

FIG. 14 shows plug 1300 fully installed in aperture 1380 in heart 1370 so that plugging structure 1306 spans aperture 1380 and frame 1302 conforms to perimeter 1398. Fingers 1308 and 1310 are engaged with opposite sides of wall 1450 to secure plug 1300. Optional markers 1314 are provided on ends 1312 of fingers 1310. In one embodiment according to the invention, markers 1314 are radiopaque. FIG. 15 shows plug 1300 as installed in wall 1350 as viewed along direction 15-15 of FIG. 14. Fingers 1308 are pressing against surface 1348 of wall 1350. Plugging structure 1306 spans the aperture in wall 1350 and is substantially flush with perimeter 1398.

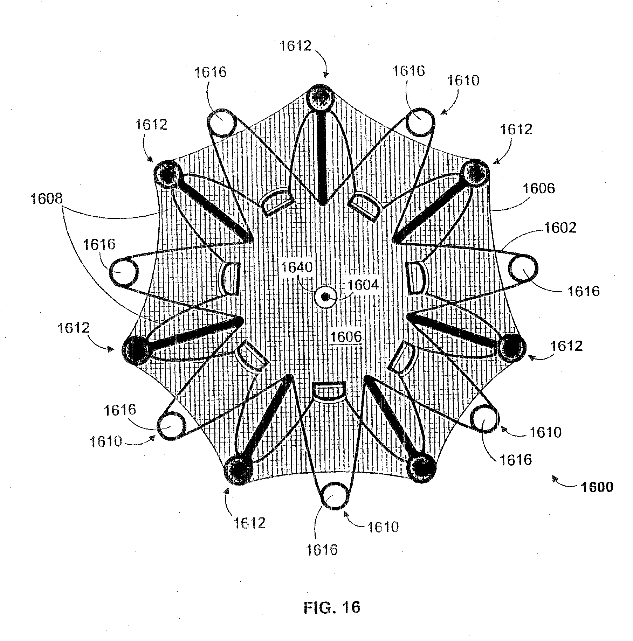

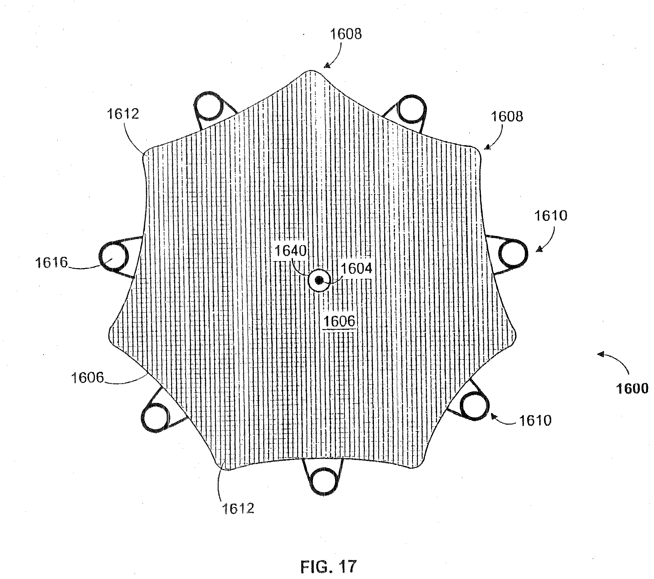

FIG. 16 shows another embodiment of a plug according to the invention. Plug 1600 includes frame 1602 which is structurally similar to frame 102 of FIG. 1. In this embodiment, however, plugging structure 1606 is supported by remote ends 1612 of fingers 1608. It will be appreciated that plugging structure could just as easily be mounted on fingers 1610. Optional marker rivets (not shown) can be placed in or near the apertures located at the of ends of fingers 1612. Optional marker rivets can also be used to attach plugging structure 1606 to frame 1602 and simultaneously provide a radiopaque marking device for locating and positioning plug 1600 using medical scanning instrumentation. Central axis 1604 passes through plugging structure 1606. Guide wire aperture 1640 allows a guide wire to be used to help control the position of plug 1600 during installation. FIG. 17 shows plug 1600 as viewed from the side opposite that shown in FIG. 16. As shown in FIG. 17, fingers 1610 and retention device receptacles 1616 can radially extend beyond plugging structure 1606.

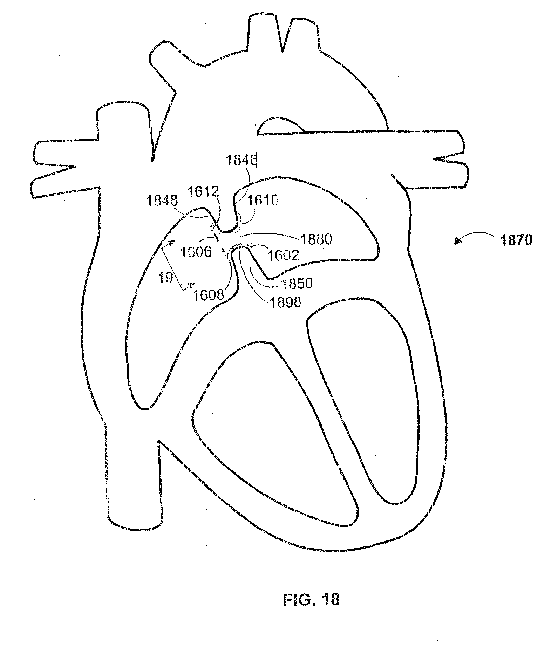

FIG. 18 shows plug 1600 fully installed in aperture 1880 of wall 1850 in patient's heart 1870. Plugging structure 1606 is attached to remote ends 1612 of fingers 1608 and is drawn against surface 1848 by fingers 1612 as fingers 1608 press against side 1848. When installed, plugging structure 1606 has a greater diameter than cavity wall aperture 1880 and thus extends beyond perimeter 1898 of aperture 1880 in order to occlude aperture 1880. Fingers 1610 engage side 1846 of wall 1850 and hold plug 1600 in position.

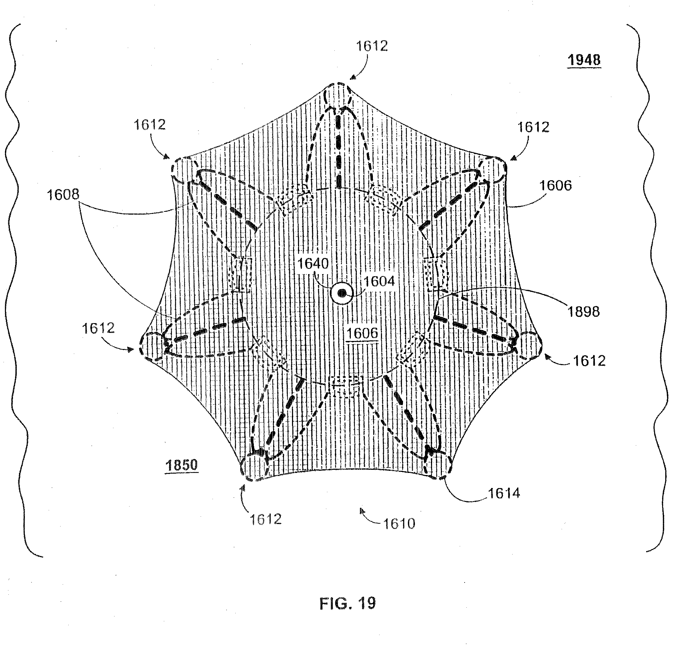

FIG. 19 shows plug 1600 as viewed from line 19-19 of FIG. 18. Fingers 1610 of frame 1602 are not shown because from this perspective they are behind wall 1850. Perimeter 1898 of aperture 1880 is shown as a broken line because it is behind plugging structure 1606. Plugging structure 1606 can be supported by rivets 1614, which are located at ends 1612.

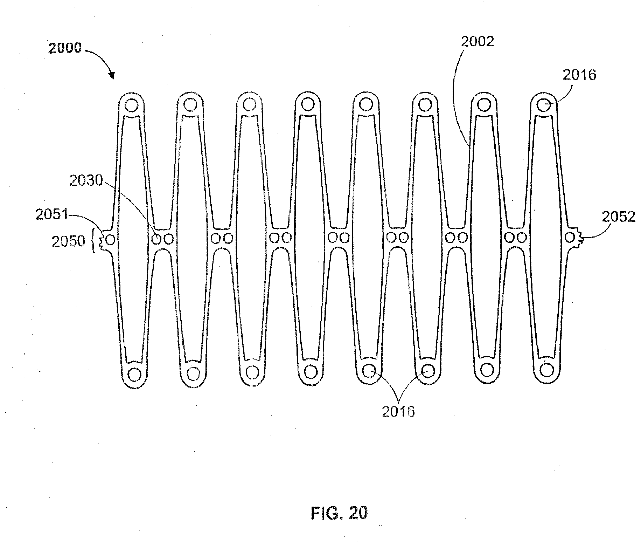

According to an exemplary form, a plug has a medial portion that defines a central passage with a central axis. To allow the medial portion to expand radially, any cross-section of that portion (and preferably the entire frame) taken perpendicularly to the central axis is substantially discontinuous. FIG. 20 shows exemplary frame 2002 in an "unfurled" state. Portions 2051 and 2052 of medial section 2050 would normally be connected so that medial section 2050 forms a perforated tube surrounding the plug's central axis. Plug 2000 can include retention device receptacles 2016 on, for example, remote ends of the fingers. Frame 2002 can also include support structures 2030 for supporting a plugging structure (not shown).

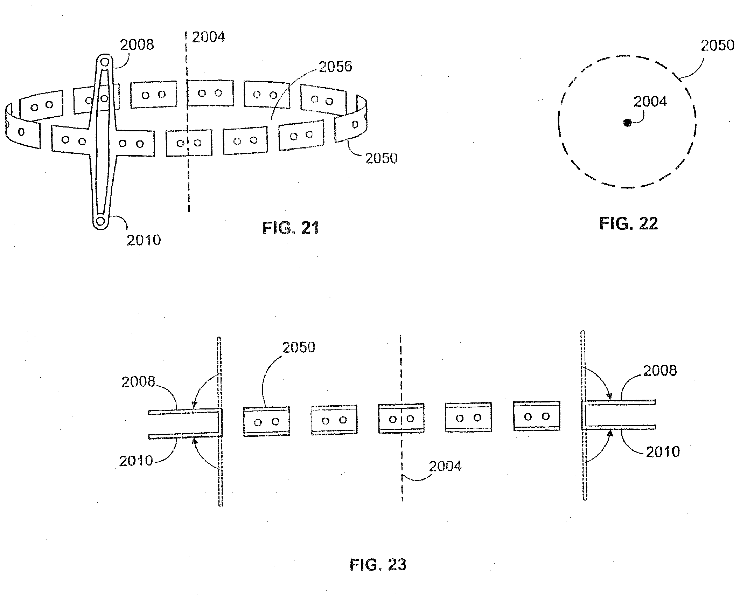

Medial section 2050, when in its operable shape, forms a perforated tubular portion. FIG. 21 is a perspective view of exemplary frame 2002 in its operable tubular shape, including particularly medial section 2050 and fingers 2008 and 2010 (remainder of fingers not shown for the sake of simplicity). Perforated tubular portion 2050 defines longitudinal passage 2056 along central axis 2004.

FIG. 22 shows a cross-sectional view of perforated tubular portion 2050 taken along a plane that is perpendicular to central axis 2004. The broken line corresponding to portion 2050 indicates that the cross section is discontinuous.

FIG. 23 shows a side view of tubular portion 2050 and selected fingers 2008 and 2010 in two different positions. As shown, fingers 2008 and 2010 are attached to the axial ends of tubular portion 2550 and are bent away from central axis 2004 into a splayed position. These fingers can be heat treated before the plug is installed to cause them to relax in this splayed position. Fingers 2008 and 2010 can also be positioned so that they point in a direction that is substantially parallel to central axis 2004 (as shown by dashed lines, which corresponds to the view of FIG. 22) for installation of the plug. This position can be achieved, for example, by inserting the plug into a delivery device.

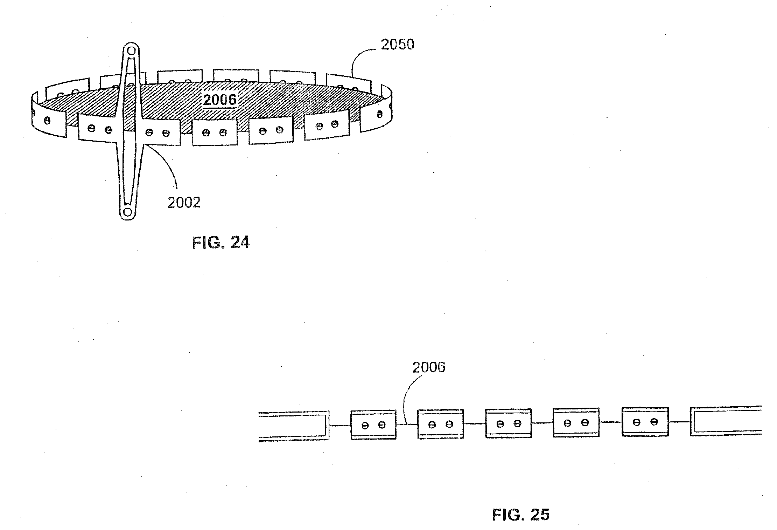

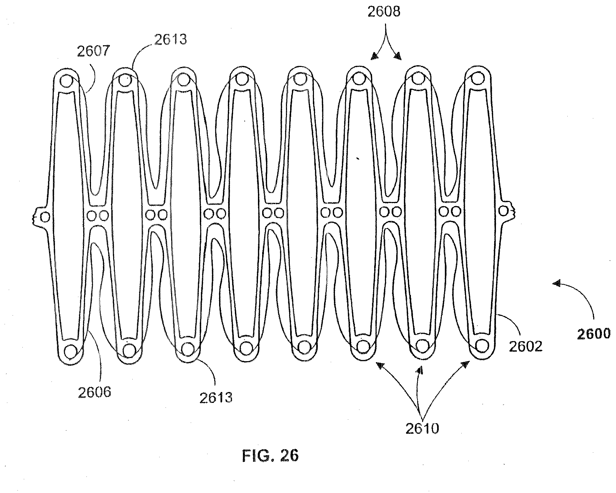

FIGS. 24 and 25 are similar to FIGS. 21 and 23, respectively, but now include plugging structure 2006 mounted to exemplary frame 2002. Alternatively, a plugging structure can be supported by support structures 2013 of fingers 2008 and/or 2010. Alternatively, plug 2000 can include two plugging structures, each of which can be supported by support structures located at opposite axial ends of plug 2000. For example, plug 2600, which includes frame 2602, is shown in FIG. 26 in an unfurled position for illustrative purposes. Frame 2602 includes first plugging structure 2606, which is mounted on support structures 2613 of fingers 2610 and second plugging structure 2607, which is mounted on support structures 2613 of fingers 2608. Support structures 2613 can also be used to mount markers or engage a retention device.

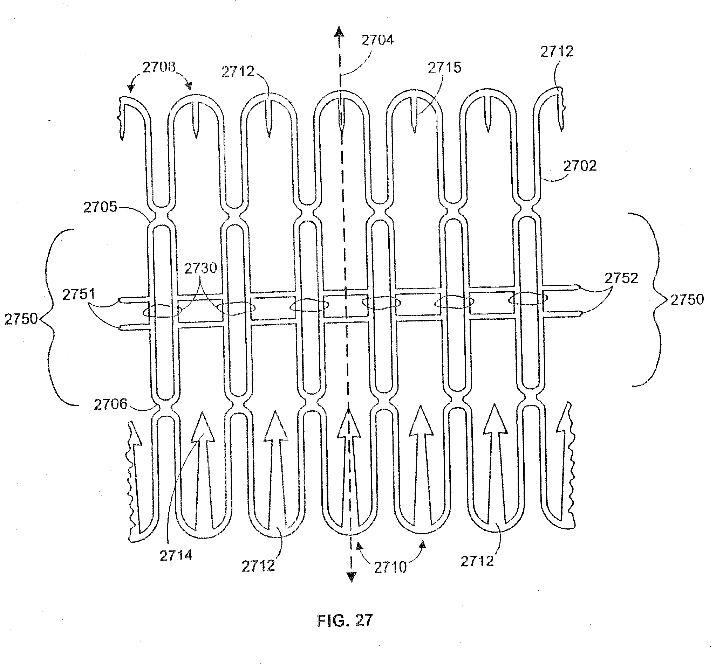

In another exemplary form, a frame having a perforated tubular portion can be formed from a plastically deformable material and can be installed using a balloon. FIG. 27, for example, shows exemplary frame 2702 in an unfurled state similar to the configuration of frame 2002 in FIGS. 20 and 26. In use, end portion 2751 and 2752 are joined to form a tubular structure surrounding central axis 2704. (Frame 2702 can be formed from a tube, thereby eliminating the need to join portions 2751 and 2752.)

When portions 2751 and 2752 are joined, medial section 2750 becomes a perforated tubular portion corresponding to perforated tubular portion 2050 shown in FIG. 22. Although the perforated tubular portion shown in FIG. 27 is axially longer than the perforated tubular portion shown in FIGS. 21 and 22, it will be appreciated that the length of the tubular portion can be matched to the thickness of the wall being plugged.

As shown in FIG. 27, fingers 2708 and fingers 2710 extend from opposite axial ends 2705 and 2706 of portion 2750, respectively. A plugging structure (not shown) can be attached, for example, to support structures 2730 to occlude the longitudinal passage during use. Piercing points 2715 and barbs 2714 are for engaging opposite sides of a wall for securing frame 2702 thereto.

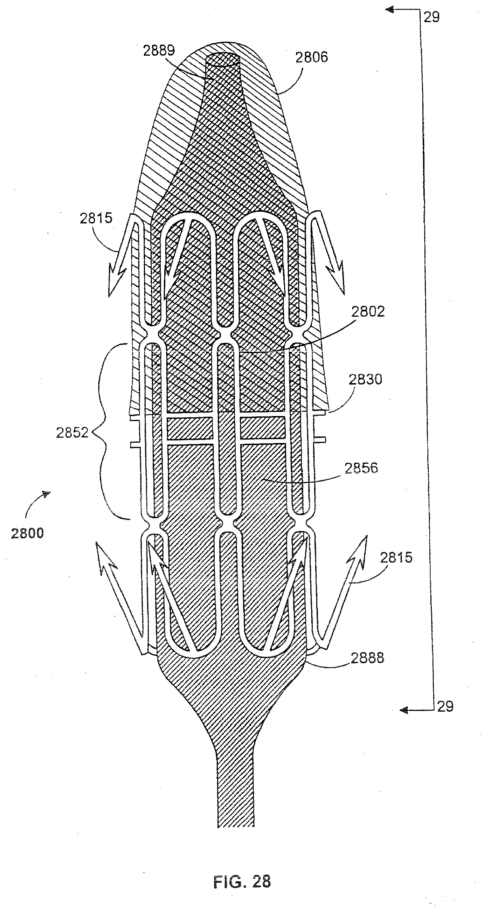

FIG. 28 shows exemplary plug 2800, which includes frame 2802 with delivery balloon 2888 inserted in longitudinal passage 2856. Delivery balloon 2888 may be inflated enough to engage frame 2802 and allow delivery of plug 2800 to a repair site inside a patient. Folded or elastic plugging structure 2806 is deflected around tip 2889 of balloon 2888. Plugging structure 2806 is attached to frame 2802 at support structure 2830. Plug 2800 can have barbs 2815, for example, for engaging a patient's body tissue.

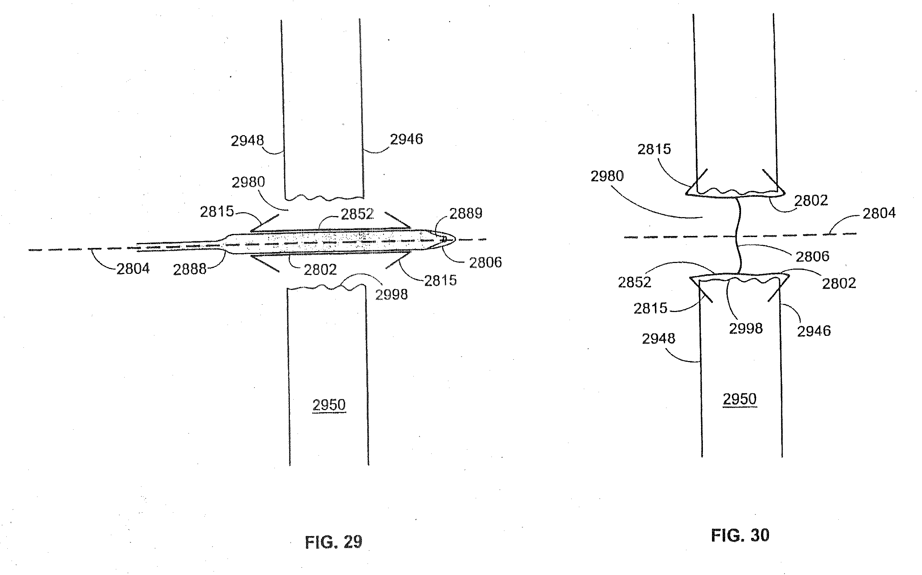

FIG. 29 shows a cross-sectional view of exemplary plug 2800 being positioned in aperture 2980 in cavity wall 2950. During installation of plug 2800, balloon 2888 engages the inside of frame 2802. Plugging structure 2806 is deflected around balloon tip 2889. Next, plug 2800 is inserted with balloon 2888 into aperture 2980. Balloon 2888 may then be inflated, causing frame 2802 to expand radially and to contract along its central axis 2804, thereby forcing barbs 2815 to pierce opposite sides 2946 and 2948 of wall 2950. This also causes perforated tubular portion 2852 to conform to perimeter 2898. If plugging structure 2806 is secured to perforated tubular portion 2852, it too is stretched or unfolded across aperture 2980.

FIG. 30 shows exemplary plug 2800 installed in aperture 2980 after frame 2802 is deformed by the expansion of balloon 2888. At this stage, balloon 2888 has been removed from perforated tubular portion 2852. The perforations (i.e., the holes) in portion 2852 allow the axial length of perforated tubular portion 2852 to decrease as its radius increases.

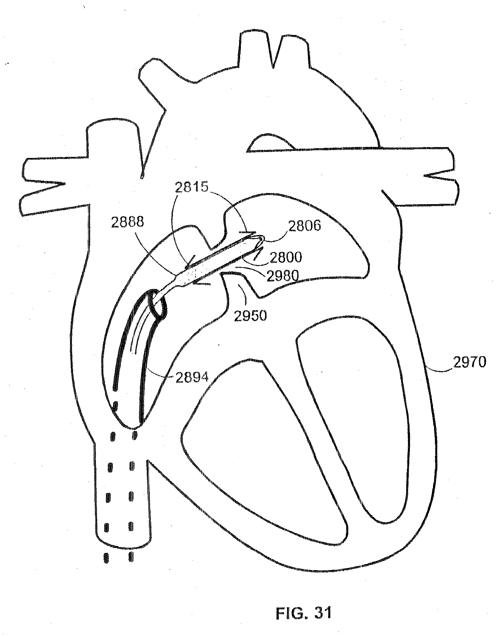

FIG. 31 shows exemplary plug 2800 being delivered to aperture 2980 in heart 2970. Plug 2800 is supported by partially inflated balloon 2888 and guided through a patient's body tissue and/or tubing via delivery guide 2894 until it is positioned with barbs 2815 on opposite sides of wall 2950.

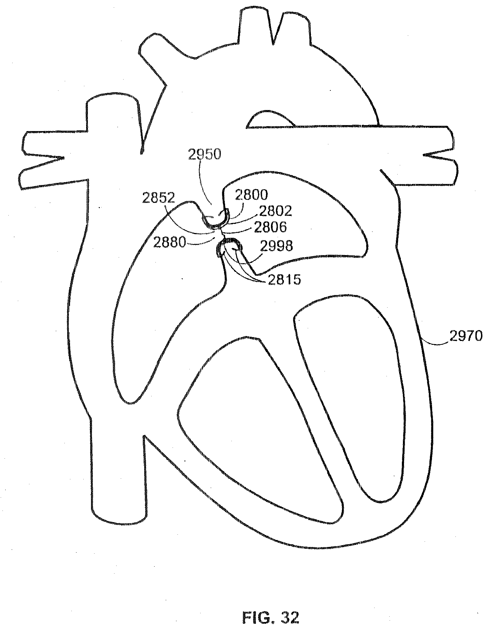

After delivery of plug 2800 to aperture 2980, plug 2800 may be fully installed by further expanding balloon 2888, thereby causing frame 2802 to deform and secure itself as shown in FIG. 32. After plug 2800 is secured, balloon 2888 is deflated and removed. When installed, tubular portion 2852 conforms to perimeter 2998 and plugging structure 2806 spans and occludes blood flow through the aperture.

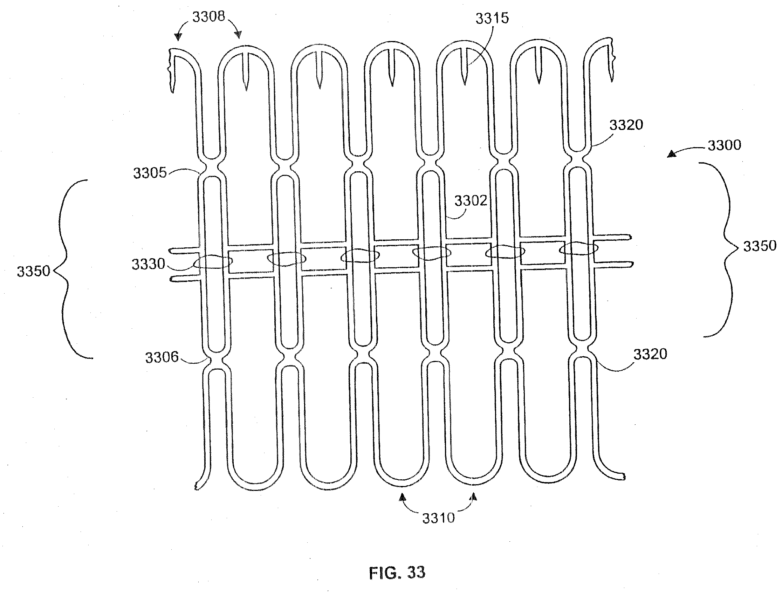

In another form an exemplary plug is disclosed for occluding a lumen of a patient's body tubing. FIG. 33 shows exemplary occlusion plug 3300, which includes frame 3302. Plug 3300 is similar to aperture plug 2700 (shown in FIG. 27), but has barbs (or points) that extend from fingers 3308 on only one axial end 3305 of medial section 3350. It will be appreciated that points 3315 are not always necessary, bus may be included in plugs where positive anchoring is desired. Frame 3302 is shown in FIG. 33 in an "unfurled" state, but as in the embodiments discussed above, would be joined to form a perforated tubular portion. Support structures 3330 can also be provided for securing a plugging structure (not shown).

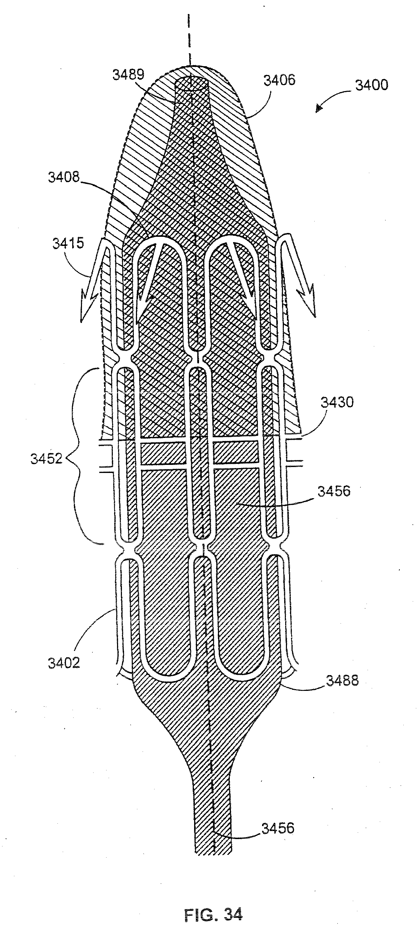

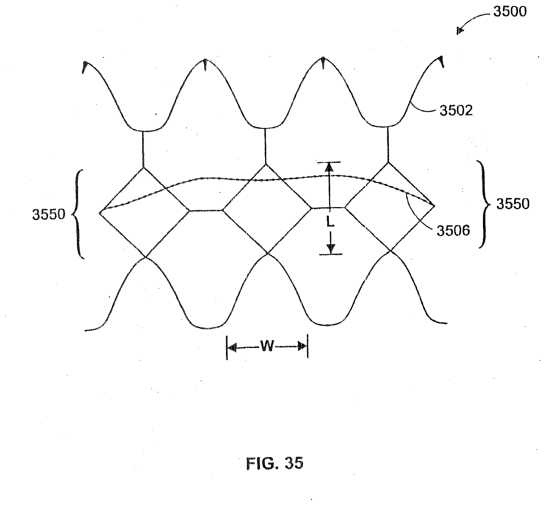

FIG. 34 shows exemplary occlusion plug 3400 mounted on partially inflated balloon 3488. Balloon 3488 occupies longitudinal passage 3456 along central axis 3456. Plugging structure 3406 is attached to frame 3402 at support structures 3430 in medial section 3452 and is deflected around tip 3489 of Balloon 3488. Piercing points 3415, which can be barbed, extend away from central axis 3456 and are destined to be embedded in the interior wall of a patient's body tubing. Exemplary occlusion plug 3500, which is shown in FIG. 35, includes a frame with a different profile from that shown in FIG. 33. FIG. 35 shows a partial view of exemplary plug 3500 in the unfurled state. Like each of the other forms discussed above, medial portion 3550 can radially expand and axially contract when a balloon is inflated therein. In particular, each of the rectangular units that make up exemplary frame 3502 can stretch, such that length 1 and width w vary inversely.

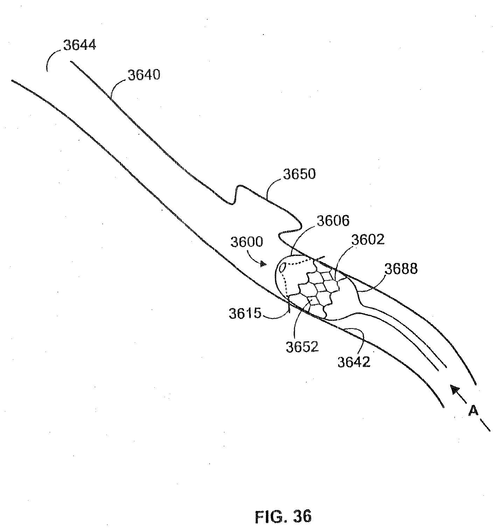

FIG. 36 shows the exemplary deployment of occlusion plug 3600 in a patient's blood vessel 3640 having aneurysm 3650. Arrow A shows the normal direction of blood flow through blood vessel 3640. Because of aneurysm 3650, it may be desirable to occlude blood vessel 3640 upstream from aneurysm 3650. As already explained above, with reference to plug 2800, for example, plug 3600 is mounted on balloon 3688 and positioned upstream on aneurysm 3650. As balloon 3688 is inflated, perforated tubular portion 3652 expands radially causing perforated tubular portion 3652 to conform to inner wall 3642 of blood vessel 3640. Plugging structure 3606 is attached to frame 3602 at points along the circumference of tubular portion 3652 and is thus stretched to occlude lumen 3644 of blood vessel 3640. As perforated tubular portion 3652 expands radially, it contracts axially and causes piercing points 3615 to engage walls 3642 and thus anchor plug 3600.

It will be understood that the foregoing is only illustrative of the principles of the invention, and that various modifications can be made by those skilled in the art without departing from the scope of the invention.

## Claims

1. A plug for closing an aperture in a wall of a patient's body cavity, said plug comprising:
(a) a frame (102) having a central axis (104) comprising:
(a1) a first plurality of fingers (108) configured to engage an interior surface of said wall of said body cavity; *and*
(a2) a second plurality of fingers (110) attached to said first plurality, wherein said second plurality is configured to engage an exterior surface of said wall of said body cavity; said pluralities of fingers (108, 110) each having remote ends (112), proximal ends (120), and lateral edges extending between said remote and proximal ends, each finger (108, 110) having a continuous surface extending between said lateral edges; wherein said pluralities of fingers are positioned substantially circumferentially with respect to said axis; and wherein any cross-section of said frame that lies in a plane substantially perpendicular to said axis is discontinuous; and
(b) a plugging structure (106) that spans said aperture when said plug is inserted in said aperture, wherein said plugging structure is attached to said frame.

2. The plug (100) of claim 1,
wherein said plugging structure (106) is supported by one of said pluralities of fingers, that may continuously, and on only one side of said wall, span said aperture when said plug is inserted in said aperture; and
wherein said pluralities of fingers (108, 110) are configured to be urged toward each other on opposite sides of said wall.

3. The plug (1600) of claim 1,
wherein said plugging structure (106) is supported by one of said pluralities of fingers (1608, 1610) and extending radially inward to said central axis (1604), and on only one side of said wall, span said aperture when said plug is inserted in said aperture; and
wherein said pluralities of fingers (1608, 1610) are configured to be urged toward each other on opposite sides of said wall.

4. The plug (1600) of claim 1, 2 or 3, wherein
(i) the frame (1602) comprises an elastic material, preferably nitinol, and/or
(ii) said plug can be detected using fluoroscopy.

5. The plug (1600) of any one of claims 1 to 4 wherein said second plurality of fingers (1610) is integral with said first plurality of fingers (1608) and preferably said first and second pluralities of fingers (1608, 1610) are formed from a unitary body.

6. The plug (1600) of any one of claims 1 to 5 wherein at least one finger of at least one of said pluralities of fingers (1608, 1610) extends substantially radially away from said central axis.

7. The plug (1600) of any one of claims 1 to 6 wherein at least one finger of at least one of said pluralities of fingers (1608, 1610) has a marker structure (114), wherein said marker structure (114) is preferably:
(i) radiopaque, or
(ii) a marker band, or
(iii) a rivet;
wherein said finger of the radiopaque marker structure has preferably an end portion and said marker band (1600) is preferably crimped to said end portion.

8. The plug (1600) of any one of claims 1 to 7 wherein at least one finger of at least one of said pluralities of fingers (1608, 1610) has a retention device receptacle (1016), wherein said retention device receptacle (1016) is preferably
(i) a locking pin aperture, or
(ii) a nose cone cover.

9. The plug (1600) of any one of claims 1 to 8 wherein at least one finger of at least one of said pluralities of fingers (1608, 1610) has
(i) a pointed end portion located remotely from said central axis (1604); and/or
(ii) a barbed end portion located remotely from said central axis (1604).

10. The plug (1600) of any one of claims 1 to 9 wherein at least one finger of at least one of said pluralities of fingers (1608, 1610) is curved, said curve being concave toward a plane perpendicular to said central axis and passing substantially between said first and second pluralities of fingers (1608, 1610).

11. The plug (1600) of any one of claims 1 to 10 wherein said fingers have end portions that are proximal to said central axis (1604) and said end portions define
(i) a substantially round cross section; and/or
(ii) a substantially elliptical cross section.

12. The plug (1600) of any one of claims 1 to 11 wherein
(i) substantially all of said fingers of at least one of said pluralities of fingers (1608, 1610) are of substantially the same length; and/or
(ii) different fingers of at least one of said pluralities of fingers (1608, 1610) have different lengths.

13. The plug (1600) of any one of claims 1 to 12 wherein at least one finger of at least one of said pluralities of fingers (1608, 1610) has
(i) a different flexural stiffness at different points along its length; and/or
(ii) a different thickness at different points along its length; and/or
(iii) a different width at different points along its length.

14. The plug (1600) of any one of claims 1 to 13 wherein at least one finger of at least one of said pluralities of fingers (1608, 1610) has a free end portion comprising an end structure configured to facilitate releasable retention of said finger by a plug delivery device (1090).

15. The plug (1600) of any one of claims 1 to 14 further comprising an elastic web between adjacent ones of said fingers; wherein said web preferably comprises silicone.

16. The plug (1600) of any one of claims 1 to 15 wherein at least one finger of at least one of said pluralities of fingers (1608, 1610) has
(i) an end portion proximal to said central axis for supporting said plugging structure (106), wherein said end portions preferably have support structures with which to affix said plugging structure (106), and/or
(ii) an end portion remote from said central axis for supporting said plugging structure (106), wherein said end portion preferably has a support structure with which to affix said plugging structure (106).

17. The plug (1600) of any one of claims 1 to 15 wherein said plugging structure (106):
(i) is elastic; and/or
(ii) can be unfolded; and/or
(iii) comprises polymeric material; and/or
(iv) comprises DACRON®; and/or
(v) comprises cloth; and/or
(vi) is sewn to said frame; and/or
(vii) has a guide wire aperture (140) through which a guide wire can pass.

18. The plug (100) of any one of claims 4 to 17,
wherein said proximal ends (120) of said pluralities of fingers (108, 110) collectively define a central space, and said plugging structure (106) extends only within said central space.

## Patentansprüche

1. Stopfen zum Verschließen einer Öffnung in einer Wand einer Körperhöhle eines Patienten, wobei der Stopfen Folgendes umfasst:
(a) einen Rahmen (102) mit einer Mittelachse (104), Folgendes umfassend:
(a1) eine erste Mehrzahl von Fingern (108), die konfiguriert ist, in eine Innenoberfläche der Wand der Körperhöhle einzugreifen; und
(a2) eine zweite Mehrzahl von Fingern (110), die an der ersten Mehrzahl befestigt ist, wobei die zweite Mehrzahl konfiguriert ist, in eine Außenoberfläche der Wand der Körperhöhle einzugreifen;
wobei die Mehrzahlen von Fingern (108, 110) jeweils entfernt angeordnete Enden (112), proximale Enden (120) und Seitenkanten, die sich zwischen entfernt angeordneten und proximalen Enden erstrecken, aufweisen, wobei jeder Finger (108, 110) eine durchgehende Oberfläche, die sich zwischen den Seitenkanten erstreckt, aufweist;
wobei die Mehrzahlen von Fingern im Wesentlichen umlaufend bezogen auf die Achse angeordnet sind; und wobei jeder Querschnitt des Rahmens, der in einer Ebene im Wesentlichen senkrecht zur Achse liegt, unterbrochen ist; und
(b) eine Stopfenstruktur (106), die die Öffnung überspannt, wenn der Stopfen in die Öffnung eingeführt ist, wobei die Stopfenstruktur an dem Rahmen befestigt ist.

2. Stopfen (100) nach Anspruch 1,
wobei die Stopfenstruktur (106) auf einer der Mehrzahlen von Fingern gelagert ist, die die Öffnung durchgehend und nur auf einer Seite der Wand überspannen kann, wenn der Stopfen in der Öffnung eingeführt ist; und
wobei die Mehrzahlen von Fingern (108, 110) konfiguriert sind, auf einander gegenüberliegenden Seiten der Wand zueinander hin gedrängt zu werden.

3. Stopfen (1600) nach Anspruch 1,
wobei die Stopfenstruktur (106) durch eine der Mehrzahlen von Fingern (1608, 1610) gelagert ist und sich radial nach innen zur Mittelachse (1604) hin und nur auf einer Seite der Wand erstreckt, die Öffnung überspannt, wenn der Stopfen in der Öffnung eingeführt ist; und
wobei die Mehrzahlen von Fingern (1608, 1610) konfiguriert sind, auf einander gegenüberliegenden Seiten der Wand zueinander gedrängt zu werden.

4. Stopfen (1600) nach Anspruch 1, 2 oder 3, wobei
(i) der Rahmen (1602) ein elastisches Material, vorzugsweise Nitinol, umfasst und/oder
(ii) der Stopfen unter Verwendung von Fluoroskopie erkannt werden kann.

5. Stopfen (1600) nach einem der Ansprüche 1 bis 4, wobei die zweite Mehrzahl von Fingern (1610) mit der ersten Mehrzahl von Fingern (1608) einstückig ausgebildet ist und vorzugsweise die erste und die zweite Mehrzahl von Fingern (1608, 1610) aus einem einteiligen Körper ausgebildet sind.

6. Stopfen (1600) nach einem der Ansprüche 1 bis 5, wobei wenigstens ein Finger von wenigstens einer der Mehrzahlen von Fingern (1608, 1610) sich im Wesentlichen radial von der Mittelachse weg erstreckt.

7. Stopfen (1600) nach einem der Ansprüche 1 bis 6, wobei wenigstens ein Finger von wenigstens einer der Mehrzahlen von Fingern (1608, 1610) eine Markerstruktur (114) aufweist, wobei die Markerstruktur (114) vorzugsweise Folgendes ist:
(i) röntgenstrahlenundurchlässig oder
(ii) ein Markerband oder
(iii) ein Niet;
wobei der Finger der röntgenstrahlenundurchlässigen Markerstruktur vorzugsweise einen Endabschnitt aufweist und das Markerband (1600) vorzugsweise zum Endabschnitt hin gekräuselt ist.

8. Stopfen (1600) nach einem der Ansprüche 1 bis 7, wobei wenigstens ein Finger von wenigstens einer der Mehrzahlen von Fingern (1608, 1610) eine Haltevorrichtungsaufnahme (1016) aufweist, wobei die Haltevorrichtungsaufnahme (1016) vorzugsweise Folgendes ist:
(i) eine Arretierstiftöffnung oder
(ii) eine Nasenkonusabdeckung.

9. Stopfen (1600) nach einem der Ansprüche 1 bis 8, wobei wenigstens ein Finger von wenigstens einer der Mehrzahlen von Fingern (1608, 1610) Folgendes aufweist:
(i) einen spitz zulaufenden Endabschnitt, der sich entfernt von der Mittelachse (1604) befindet; und/oder
(ii) einen mit Stacheln versehenen Endabschnitt, der sich entfernt von der Mittelachse (1604) befindet.

10. Stopfen (1600) nach einem der Ansprüche 1 bis 9, wobei wenigstens ein Finger von wenigstens einer der Mehrzahlen von Fingern (1608, 1610) gewölbt ist, wobei die Wölbung zu einer Ebene senkrecht zur Mittelachse konkav ist und im Wesentlichen zwischen der ersten und der zweiten Mehrzahl von Fingern (1608, 1610) verläuft.

11. Stopfen (1600) nach einem der Ansprüche 1 bis 10, wobei die Finger Endabschnitte aufweisen, die proximal zur Mittelachse (1604) sind und die Endabschnitte Folgendes definieren:
(i) einen im Wesentlichen runden Querschnitt; und/oder
(ii) einen im Wesentlichen elliptischen Querschnitt.

12. Stopfen (1600) nach einem der Ansprüche 1 bis 11, wobei
(i) im Wesentlichen alle der Finger von wenigstens einer der Mehrzahlen von Fingern (1608, 1610) im Wesentlichen die gleiche Länge aufweisen; und/oder
(ii) verschiedene Finger von wenigstens einer der Mehrzahlen von Fingern (1608, 1610) verschiedene Längen aufweisen.

13. Stopfen (1600) nach einem der Ansprüche 1 bis 12, wobei wenigstens ein Finger von wenigstens einer der Mehrzahlen von Fingern (1608, 1610) Folgendes aufweist:
(i) eine unterschiedliche Biegesteifigkeit an verschiedenen Punkten entlang seiner Länge; und/oder
(ii) eine unterschiedliche Dicke an verschiedenen Punkten entlang seiner Länge; und/oder
(iii) eine unterschiedliche Breite an verschiedenen Punkten entlang seiner Länge.

14. Stopfen (1600) nach einem der Ansprüche 1 bis 13, wobei wenigstens ein Finger von wenigstens einer der Mehrzahlen von Fingern (1608, 1610) einen freien Endabschnitt aufweist, der eine Endstruktur, die konfiguriert ist, lösbares Halten des Fingers durch eine Stopfeneinführvorrichtung (1090) zu erleichtern, umfasst.

15. Stopfen (1600) nach einem der Ansprüche 1 bis 14, ferner umfassend eine elastische Bahn zwischen nebeneinanderliegenden der Finger; wobei die Bahn vorzugsweise Silicon umfasst.

16. Stopfen (1600) nach einem der Ansprüche 1 bis 15, wobei wenigstens ein Finger von wenigstens einer der Mehrzahlen von Fingern (1608, 1610) Folgendes aufweist:
(i) einen Endabschnitt proximal zur Mittelachse zum Lagern der Stopfenstruktur (106), wobei die Endabschnitte vorzugsweise Lagerstrukturen aufweisen, mit denen die Stopfenstruktur (106) zu befestigen ist, und/oder
(ii) einen Endabschnitt entfernt von der Mittelachse zum Lagern der Stopfenstruktur (106), wobei der Endabschnitt vorzugsweise eine Lagerstruktur, mit der die Stopfenstruktur (106) zu befestigen ist, aufweist.

17. Stopfen (1600) nach einem der Ansprüche 1 bis 15, wobei die Stopfenstruktur (106)
(i) elastisch ist; und/oder
(ii) entfaltet werden kann; und/oder
(iii) Polymermaterial umfasst; und/oder
(iv) DACRON® umfasst; und/oder
(v) Gewebe umfasst; und/oder
(vi) an den Rahmen geheftet ist; und/oder
(vii) eine Führungsdrahtöffnung (140) aufweist, durch die ein Führungsdraht durchlaufen kann.

18. Stopfen (100) nach einem der Ansprüche 4 bis 17,
wobei die proximalen Enden (120) der Mehrzahlen von Fingern (108, 110) gemeinsam einen mittigen Raum definieren und die Stopfenstruktur (106) sich nur innerhalb des mittigen Raums erstreckt.

## Revendications

1. Bouchon pour fermer une ouverture dans une paroi d'une cavité corporelle d'un patient, ledit bouchon comprenant :
(a) un cadre (102) ayant un axe central (104) comprenant :
(a1) une première pluralité de doigts (108) configurée pour engager une surface intérieure de ladite paroi de ladite cavité corporelle ; et
(a2) une deuxième pluralité de doigts (110) fixée à ladite première pluralité, où ladite deuxième pluralité est configurée pour engager une surface extérieure de ladite paroi de ladite cavité corporelle ; lesdites pluralités de doigts (108, 110) ayant chacune des extrémités distantes (112), des extrémités proximales (120) et des bords latéraux s'étendant entre lesdites extrémités distantes et proximales, chaque doigt (108, 110) ayant une surface continue s'étendant entre lesdits bords latéraux ; où lesdites pluralités de doigts sont positionnées de manière sensiblement circonférentielle par rapport audit axe ; et où une quelconque section transversale dudit cadre qui se situe dans un plan sensiblement perpendiculaire audit axe est discontinue ; et
(b) une structure d'obturation (106) qui recouvre ladite ouverture lorsque ledit bouchon est inséré dans ladite ouverture, où ladite structure d'obturation est fixée audit cadre.

2. Bouchon (100) selon la revendication 1,
dans lequel ladite structure d'obturation (106) est soutenue par l'une desdites pluralités de doigts, qui peut recouvrir de façon continue, et sur un seul côté de ladite paroi, ladite ouverture lorsque ledit bouchon est inséré dans ladite ouverture ; et
dans lequel lesdites pluralités de doigts (108, 110) sont configurées pour être serrées entre elles sur les côtés opposés de ladite paroi.

3. Bouchon (1600) selon la revendication 1,
dans lequel ladite structure d'obturation (106) est soutenue par l'une desdites pluralités de doigts (1608, 1610) et recouvre par extension radiale vers l'intérieur par rapport audit axe central (1604), et sur un seul côté de ladite paroi, ladite ouverture lorsque ledit bouchon est inséré dans ladite ouverture ; et
dans lequel lesdites pluralités de doigts (1608, 1610) sont configurées pour être serrées entre elles sur les côtés opposés de ladite paroi.

4. Bouchon (1600) selon la revendication 1, 2 ou 3, dans lequel
(i) le cadre (1602) comprend un matériau élastique, de préférence du nitinol, et/ou
(ii) ledit bouchon peut être détecté par fluoroscopie.

5. Bouchon (1600) selon l'une quelconque des revendications 1 à 4, dans lequel ladite deuxième pluralité de doigts (1610) est intégrée dans ladite première pluralité de doigts (1608) et de préférence lesdites première et deuxième pluralités de doigts (1608, 1610) sont formées à partir d'un corps unitaire.

6. Bouchon (1600) selon l'une quelconque des revendications 1 à 5, dans lequel au moins un doigt d'au moins une desdites pluralités de doigts (1608, 1610) s'étend sensiblement radialement à partir dudit axe central.

7. Bouchon (1600) selon l'une quelconque des revendications 1 à 6, dans lequel au moins un doigt d'au moins une desdites pluralités de doigts (1608, 1610) a une structure de marquage (114), dans lequel ladite structure de marquage (114) est de préférence :
(i) radio-opaque, ou
(ii) une bande de marquage, ou
(iii) un rivet ;
dans lequel ledit doigt de la structure de marquage radio-opaque a de préférence une partie terminale et ladite bande de marquage (1600) est de préférence sertie sur ladite partie terminale.

8. Bouchon (1600) selon l'une quelconque des revendications 1 à 7, dans lequel au moins un doigt d'au moins une desdites pluralités de doigts (1608, 1610) a un réceptacle pour dispositif de retenue (1016), dans lequel ledit réceptacle pour dispositif de retenue (1016) est de préférence
(i) une ouverture à goupille de sécurité, ou
(ii) un couvercle conique.

9. Bouchon (1600) selon l'une quelconque des revendications 1 à 8, dans lequel au moins un doigt d'au moins une desdites pluralités de doigts (1608, 1610) a
(i) une partie terminale pointue située à distance dudit axe central (1604) ; et/ou
(ii) une partie terminale indentée située à distance dudit axe central (1604).

10. Bouchon (1600) selon l'une quelconque des revendications 1 à 9, dans lequel au moins un doigt d'au moins une desdites pluralités de doigts (1608, 1610) est incurvé, ladite courbure étant concave par rapport à un plan perpendiculaire audit axe central et passant sensiblement entre lesdites première et deuxième pluralités de doigts (1608, 1610).

11. Bouchon (1600) selon l'une quelconque des revendications 1 à 10, dans lequel lesdits doigts ont des parties terminales qui sont proximales par rapport audit axe central (1604) et lesdites parties terminales définissent
(i) une section transversale sensiblement ronde ; et/ou
(ii) une section transversale sensiblement elliptique.

12. Bouchon (1600) selon l'une quelconque des revendications 1 à 11, dans lequel
(i) sensiblement tous lesdits doigts d'au moins une desdites pluralités de doigts (1608, 1610) sont sensiblement de la même longueur ; et/ou
(ii) différents doigts d'au moins une desdites pluralités de doigts (1608, 1610) ont différentes longueurs.

13. Bouchon (1600) selon l'une quelconque des revendications 1 à 12, dans lequel au moins un doigt d'au moins une desdites pluralités de doigts (1608, 1610) a
(i) une rigidité en flexion différente à différents points sur sa longueur ; et/ou
(ii) une épaisseur différente à différents points sur sa longueur ; et/ou
(iii) une largeur différente à différents points sur sa longueur.

14. Bouchon (1600) selon l'une quelconque des revendications 1 à 13, dans lequel au moins un doigt d'au moins une desdites pluralités de doigts (1608, 1610) a une partie terminale libre comprenant une structure terminale configurée pour faciliter la retenue libérable dudit doigt par un dispositif de distribution de bouchons (1090).

15. Bouchon (1600) selon l'une quelconque des revendications 1 à 14, comprenant en outre une toile élastique entre les adjacents desdits doigts ; dans lequel ladite toile comprend de préférence de la silicone.

16. Bouchon (1600) selon l'une quelconque des revendications 1 à 15, dans lequel au moins un doigt d'au moins une desdites pluralités de doigts (1608, 1610) a
(i) une partie terminale proximale par rapport audit axe central pour soutenir ladite structure d'obturation (106), ladite partie terminale ayant de préférence des structures de support avec lesquelles fixer ladite structure d'obturation (106), et/ou
(ii) une partie terminale à distance dudit axe central pour soutenir ladite structure d'obturation (106), ladite partie terminale ayant de préférence une structure de support avec laquelle fixer ladite structure d'obturation (106).

17. Bouchon (1600) selon l'une quelconque des revendications 1 à 15, dans lequel ladite structure d'obturation (106) :
(i) est élastique ; et/ou
(ii) peut être dépliée ; et/ou
(iii) comprend un matériau polymère ; et/ou
(iv) comprend du DACRON® ; et/ou
(v) comprend du tissu ; et/ou
(vi) est cousue audit cadre ; et/ou
(vii) a une ouverture pour fil guide (140) à travers laquelle un fil guide peut passer.

18. Bouchon (100) selon l'une quelconque des revendications 4 à 17,
dans lequel lesdites extrémités proximales (120) desdites pluralités de doigts (108, 110) définissent ensemble un espace central, et ladite structure d'obturation (106) s'étend seulement à l'intérieur dudit espace central.
